# EUROPÄISCHE PATENTSCHRIFT

(11) **EP 0 956 851 B1**
(45) Veröffentlichungstag und Bekanntmachung des Hinweises auf die Patenterteilung: **08.03.2006**
(21) Anmeldenummer: 99810382.4
(22) Anmeldetag: 04.05.1999
(51) Int. Cl.: A61Q 5/00, A61Q 19/00, A61Q 17/00, A61Q 19/10

(54) **Verwendung von Nanodispersionen in kosmetischen Endformulierungen**
Use of nanotopes in cosmetic products
Utilisation des nanotopes comme composants des produits cosmétiques

(30) Priorität: 11.05.1998 EP 98810421
(43) Veröffentlichungstag der Anmeldung: 17.11.1999
(73) Patentinhaber: Ciba Specialty Chemicals Holding Inc., 4057 Basel (CH); VESIFACT AG, 6340 Baar (CH)
(72) Erfinder: Hüglin, Dietmar, 79591 Eimeldingen (DE); Röding, Joachim Friedrich, 79410 Badenweiler (DE); Supersaxo, Andreas Werner, 6340 Baar (CH); Weder, Hans Georg, 8803 Rüschlikon (CH)

(56) Entgegenhaltungen:
- EP-A- 0 349 150
- EP-A- 0 711 557
- EP-A- 0 852 941
- WO-A-96/37192
- WO-A-97/03642
- WO-A-97/21428
- PATENT ABSTRACTS OF JAPAN vol. 97, no. 007, 31. Juli 1997 (1997-07-31) & JP 09 059145 A (TAISHO PHARMACEUT. CO. LTD.), 4. März 1997 (1997-03-04)

## Beschreibung

Die vorliegende Erfindung betrifft die Verwendung von bestimmten Nanodispersionen in kosmetischen Endformulierungen, kosmetische Endformulierungen, enthaltend diese Nanodispersionen sowie die verschiedenen kosmetischen Verwendungen dieser Endformulierungen.

Kosmetische Präparate enthalten neben ihren Grundstoffen, die für die Bildung der kosmetischen Formulierung verantwortlich sind, weitere funktionelle Wirksubstanzen. Diese werden den kosmetischen Grundformulierungen zugegeben und dienen z.B. zur Pflege, zum Schutz, zur Färbung, zur Reinigung, zur Desinfizierung, zur Erhöhung der Feuchtigkeit in der Haut, zur Regeneration und zur Aktivierung der Haut oder der Haare.

Damit diese Stoffe ihre Wirkung an der gewünschten Stelle entfalten können, müssen sie an den jeweiligen Ort, wie z.B. die Oberfläche der Haut, Schleimhaut, Nägeln, Zahnschmelz oder Haar, aber auch in epidermate und dermale Bereiche der Haut mittels sogenannter Träger- und Transportvehikel (Carriersysteme) transportiert werden. Zahlreiche kosmetische Wirkstoffe, wie z.B. wasserlöslichen Vitamine, die Gruppe der Aminosäuren oder die wasserlöslichen Melanine, werden dazu in Liposomen verkapselt. Lipophile Substanzen können, bedingt durch die Eigenschaften von Liposomen, nur in geringen Mengen in die lipophilen Bereiche der Liposomenmembran eingeschlossen werden. Deshalb werden für solche Verbindungen, wie z.B. fettlösliche Vitamine, sogenannte Nanoemulsionen, auch Nanopartikel genannt, eingesetzt.

Der Verwendung dieser genannten Carriersysteme in üblichen kosmetischen Formulierungen sind jedoch, bedingt durch die physikalischen und chemischen Eigenschaften der Membranen und der membranbildenden Moleküle, enge Grenzen gesetzt.

Herkömmlich aufgebaute Membranen sind gegenüber amphiphilen Substanzen, wie z.B. ionogenen oder nicht-ionogenen Emulgatoren, Fettaminen, amphoteren Emulgatoren, waschaktiven Substanzen, Verdickungsmittel oder Konservierungsmittel, welche die Grundlage für viele kosmetische Präparate darstellen oder als sogenannte Wirkstoffe oder Hilfsstoffe in kosmetischen Endformulierungen enthalten sind, sehr empfindlich. In Anwesenheit solcher Substanzen wird die Struktur der Membranen verändert, was zu einer Zerstörung des zugegebenen Carriersystems in der Zubereitung führen kann.

Übliche Membranen sind auch dem Angriff von Säuren und Basen ausgesetzt. So ist es beispielsweise erforderlich, daß man Phospholipide enthaltende Carriersysteme nur im Bereich des sogenannten pH-Optimums , der bei einem pH-Wert von ca. 6,5 liegt, einsetzen kann. Ein Abfallen oder Ansteigen des pH-Wertes führt zur Hydrolyse der membranbildenden Moleküle.

Überraschenderweise wurde nun gefunden, dass Nanodispersionen geeigneter Zusammensetzung in Gegenwart von amphiphilen Substanzen in kosmetische Endformulierungen über einen breiten pH-Bereich auf sehr einfache Weise unter Wahrung ihrer morphologischen und physikalisch-chemischen Eigenschaften eingearbeitet werden können.

Gegenstand der vorliegenden Erfindung ist daher die Verwendung einer Nanodispersion, enthaltend
(a) 0.1 - 30 Gew-% eines Phospolipid,
(b) 1 - 50 Gew-% polyethoxylierte Fettalkohole, polyethoxylierte Fettsäuren, polyethoxylierte Vitamin E Derivate, polyethoxyliertes Lanolin und dessen Derivate, polyethoxylierte Fettsäurepartialglyceride, polyethoxylierte Alkylphenole, Schwefelsäurehalbester polyethoxylierter Fettalkohole und deren Salze, polyethoxylierte Fettamine und Fettsäureamide, polyethoxylierte Kohlenhydrate, Blockpolymerisate von Ethylenoxid und Propylenoxid
(c) 0.1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen kosmetischen Wirkstoffes oder eine Mischung dieser Stoffe, und
(d) einen C₂-C₈-Alkohol

erhältlich durch
(α) Mischen der Komponenten (a), (b), (c) und (d) mit gewöhnlichen Rührapparaten bis eine homogene, klare Flüssigkeit entsteht, und
(β) Zugabe der im Schritt (α) erhaltenen Flüssigkeit in die Wasserphase, in kosmetischen Endformulierungen.

Der Schritt (α) erfolgt gewöhnlich bei Raumtemperatur, gegebenenfalls unter Erwärmung und unter Normaldruckbedingungen. Das Mischen geschieht mit gewöhnlichen Rührapparaten, wie z.B. Propeller-, Schrägblatt- oder Magnetrührwerken und ohne die Zuhilfenahme von speziellen mechanischen Rührhilfen.

Das Mischen der Komponenten (a), (b) und (c) (= Schritt (α)) erfolgt in wasserfreiem Medium, d.h. der Zusatz von Wasser ist nicht notwendig.

Der Schritt (β) erfolgt durch Zugabe der im Schritt (α) erhaltenen Flüssigkeit, der Nanodispersions-Vorphase, in die Wasserphase der kosmetischen Endformulierungen. Durch die besondere Auswahl der Komponenten (a), (b) und (c) entstehen dabei unmittelbar ultrafeine, monodisperse Nanodispersionen. Dabei kann auf eine Homogenisierung mittels Düsen-, Rotor-Stator- oder Ultraschallhomogenisatoren verzichtet werden, die normalerweise dazu dient, grob- oder zumindest heterodisperse Systeme in feine monodisperse Systeme zu berführen. Der Schritt (β) ist daher gekennzeichnet durch die Abwesenheit von hohen Scher- oder Kavitationskräften.

Der Schritt (β) wird gewöhnlich bei Raumtemperatur durchgeführt, die im Bereich der jeweiligen ÖI/Wasser- Phaseninversionstemperatur (PIT) liegt.

Die durch die Verfahrensschritte (α) und (β) charakterisierten Nanodispersionen weisen Partikel mit einem mittleren Durchmesser von <50 nm, typischerweise kleiner 30 nm, auf. Die Verteilung ist monodispers und gehorcht einer Gaus-Verteilung.

Ganz besonders bevorzugt ist dabei ein Phospholipid der Formel 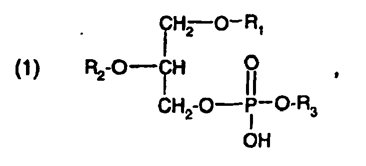
worin
- R₁: C₁₀-C₂₀-Acyl;
- R₂: Wasserstoff oder C₁₀-C₂₀-Acyl
- R₃: Wasserstoff, 2-Trimethylamino-1-ethyl, 2-Amino-1-ethyl, nicht substituiertes oder durch eine oder mehrere Carboxy-, Hydroxy- oder Amino-Gruppen substituiertes C₁-C₅-Alkyl; die Inositol- oder die Glycerylgruppe;

bedeuten; oder Salze dieser Verbindungen.

C₁₀-C₂₀-Acyl ist vorzugsweise geradkettiges C₁₀-C₂₀-Alkanoyl mit einer geraden Anzahl an C-Atomen und geradkettiges C₁₀-C₂₀-Alkenoyl mit einer Doppelbindung und einer geraden Anzahl an C-Atomen.

Geradkettiges C₁₀-C₂₀-Alkanoyl mit einer geraden Anzahl an C-Atomen sind beispielsweise n-Dodecanoyl, n-Tetradecanoyl, n-Hexadecanoyl oder n-Octadecanoyl.

Geradkettiges C₁₀-C₂₀-Alkenoyl mit einer Doppelbindung und einer geraden Anzahl an C-Atomen sind beispielsweise 6-cis- oder-6-trans-, 9-cis- oder 9-trans- Dodecenoyl, -Tetradecenoyl, -Hexadecenoyl, -Octadecenoyl oder -Icosenoyl, insbesondere 9-cis-Octadecenoyl (Oleoyl), femer 9,12-cis-Octadecadienoyl oder 9,12,15-cis-octadecatrienoyl.

Ein Phospholipid der Formel (1), worin R₃ 2-Trimethylamino-1-ethyl bedeutet, wird mit dem Trivialnamen Lecithin und ein Phospholipid der Formel (1), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen oder Mischungen davon.

Das Phospholipid der Formel (1) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter vom definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Phospholipide, welche bezüglich R₁ und R₂ keine einheitliche Zusammensetzung haben, werden als "natürlich vorkommende" Phospholipide definiert. Der Begriff Solche natürlichen Phospholipide sind ebenfalls Lecithine und Kephaline, deren Acylgruppen R₁ und R₂ von natürlich vorkommenden Fettsäuregemischen abgeleitet sind.

Die Forderung "im wesentlichen reines" Phospholipid der Formel (1) definiert einen Reinheitsgrad von mehr als 90 Gew.-%, vorzugsweise mehr als 95 Gew.-% des Phospholipids der Formel (1), welcher anhand geeigneter Bestimmungsmethoden, z.B. papier- oder dünnschichtchromatographisch, mit HPLC oder enzymatischem Farbtest, nachweisbar ist.

In einem Phospholipid der Formel (1) ist R₃ mit der Bedeutung C₁-C₄-Alkyl beispielsweise Methyl oder Ethyl. Die Bedeutung Methyl ist bevorzugt.

R₃ mit den Bedeutungen "durch eine oder mehrere Carboxy-, Hydroxy- oder Amino-Gruppen substituiertes C₁-C₅-Alkyl" sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl, 3-Amino-3-carboxy-n-propyl oder 2-Amino-2-carboxy-n-propyl, vorzugsweise 2-Amino-2-carboxyethyl.

Phospholipide der Formel (1) mit diesen Gruppen können in Salzform, z.B. als Natrium- oder Kaliumsalz, vorliegen.

Phospholipide der Formel (1), worin R₃ die Inositol- oder die Glycerylgruppe bedeutet, sind unter den Bezeichnungen Phosphatidylinositol und Phosphatidylglycerol bekannt.

Für die Acylreste in den Phospholipiden der Formel (1) sind auch die in Klammem angegebenen Bezeichnungen gebräuchlich:
9-cis-Dodecenoyl (Lauroleoyl), 9-Cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-Icosenoyl (Gadoleoyl), 5,8,11,14-cis-Eicosatetraenoyl (Arachidonoyl), n-Dodecanoyl, (Lauroyl), n-Tetradecanoyl (Myristoyl), n-Hexadecanoyl (Palmitoyl), n-Octadecanoyl (Stearoyl), n-Icosanoyl (Arachidoyl), n-Docosanoyl (Behenoyl), n-Tetracosanoyl (Lignoceroyl).

Ein Salz des Phospholipids der Formel (1) ist vorzugsweise kosmetisch annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen der Qualität LIPOID S 100 oder S 75 oder ein Lecithin definiert in der Monographie USP23/NF 18.

Emulgatoren vom Polyoxyethylen-Typ sind ganz besonders bevorzugt. Beispiele solcher Emulgatoren sind:
- Polyethoxylierte Fettalkohole wie z.B. Oleth-20;
- Polyethoxylierte Fettsäuren wie z.B. Polyoxyl 20 Stearat;
- Polyethoxyliertes Vitamin E Derivate wie z.B. Vitamin E Polyethylene Glycol 1000 Succinat;
- Polyethoxylierte Lanolin und Lanolin Derivate wie z.B. Laneth-20;
- Polyethoxylierte Fettsäurepartialglyceride wie z.B. Diethylenglykolmonostearat;
- Polyethoxylierte Alkylphenole wie z.B. Ethylphenolpoly(ethylenglykolether)11;
- Schwefelsäurehalbester polyethoxylierter Fettalkohole und deren Salze wie z.B. C₁₂-C₁₄₋Fettalkoholethersulfat-2 EO-Natriumsalz;
- Polyethoxylierte Fettamine und Fettsäureamide;
- Polyethoxylierte Kohlenhydrate
- Blockpolymerisate von Ethylenoxid und Propylenoxid, wie z.B. Poloxamer 188.

Besonders bevorzugt wird als Komponente (c) ein Sonnenschutzfilter oder ein fettlösliches Vitamin verwendet.

Ein für die Hautkosmetik geeigneter Wirkstoff, eine Wirkstoffzusammensetzung oder ein Wirkstoffextrakt ist ein Inhaltsstoff oder ein Gemisch von Inhaltsstoffen, welches für die dermale oder topische Verabreichung zugelassen ist.
Beispielhaft seien aufgeführt:
- Wirkstoffe, die eine reinigende Wirkung an der Hautoberfläche und den Haaren bewirken. Hierzu zählen alle Substanzen, die der Hautreinigung dienen, wie Öle, Seifen, Syndets und feste Stoffe;
- Wirkstoffe mit deodorierender und schweisshemmender Wirkung: hierzu zählen Antiperspirantien auf Basis von Aluminium- oder Zinksalzen, Deodorantien, die bakterizide, bzw. bakteriostatische deodoriende Substanzen, wie z.B. Triclosan, Hexachlorophen, Alkohole und kationaktive Substanzen enthalten, wie z.B. quaternäre Ammoniumsalze und Geruchsabsorber, wie z.B. ®Grillocin (Kombination von Zinkrizinoleat und verschiedenen Zusätzen) oder Triethylzitrat, gegebenenfalls in Kombination mit einem Antioxidans, wie z.B. Butylhydroxytoluol) oder lonenaustauschharze;
- Wirkstoffe, die einen Schutz gegen Sonnenlicht bieten (UV-Filter): geeignete Wirkstoffe sind Filtersubstanzen ("sunscreens''), die UV-Strahlung aus dem Sonnenlicht absorbieren und in Wärme umwandeln können. Je nach der gewünschten Wirkung sind folgende Lichtschutzmittel bevorzugt: Lichtschutzmittel, die selektiv Sonnenbrand erzeugende energiereiche UV-Strahlung im Bereich von ca. 280-315 nm absorbieren (UV-B-Absorber) und den längerwelligen Bereich von ca. 315-400 nm (UV-A-Bereich) transmittieren, sowie Lichtschutzmittel, welche nur die längerwellige Strahlung des UV-A-Bereichs von 315-400 nm absorbieren (UV-A-Absorber).
   Geeignete Lichtschutzmittel sind z.B. organische UV-Absorber aus der Klasse der p-Aminobenzoesäurederivate, Salicylsäurederivate, Benzophenonderivate, Dibenzoylmethanderivate, Diphenylacrylatderivate, Benzofuranderivate, polymere UV-Absorber, enthaltend eine oder mehrere silizium-organische Reste, Zimtsäurederivate, Campherderivate, Trianilino-s-Triazinderivate, Phenylbenzimidazolsulfonsäure und deren Salze, Menthyl-Anthranilate, Benzotriazolderivate, und/oder ein anorganisches Mikropigment ausgewählt aus mit Aluminiumoxid oder Siliciumdioxid umhülltem TiO₂, Zinkoxid oder Mica.
   Beispielhafte Verbindungen für p-Aminobenzoesäurederivate:
   4-Aminobenzoesäure (PABA); Ethyldihydroxypropyl-PABA der Formel 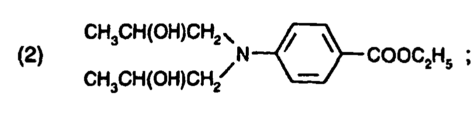 PEG-25-PABA der Formel 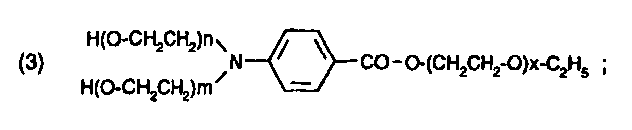
      worin m, n und x dieselbe Bedeutung haben und je höchstens 25 bedeuten;
   Octyldimethyl PABA der Formel 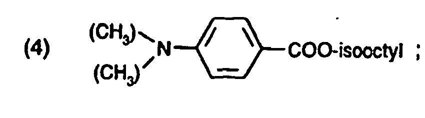 oder Glycylaminobenzoat der Formel

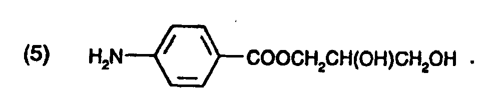

   Beispielhafte Verbindungen für Salicylsäurederivate:
   Homomenthylsalicylat der Formel 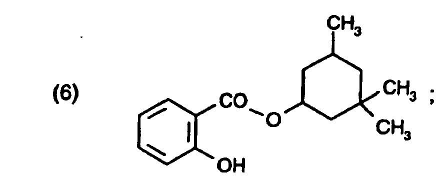 Triethanolaminsalicylat der Formel 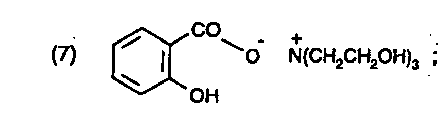 Amyl-p-dimethylaminobenzoat der Formel 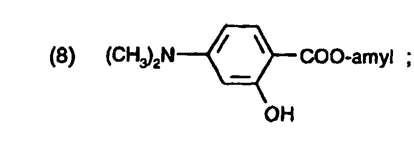 Octylsalicylat der Fomel 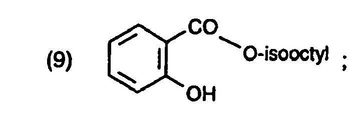 oder 4-Isopropylbenzylsalicylat der Formel

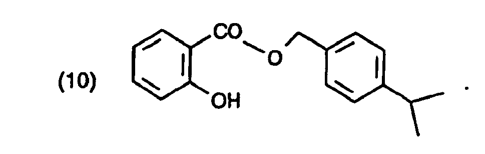

   Beispielhafte Verbindungen für Benzophenonderivate:
   Benzophenon-3-(2-hydroxy-4-methoxybenzophenon), Benzophenon-4-(2-hydroxy-4-methoxybenzophenon-5-sulfonsäure) oder Benzophenon-8-(2,2'-dihydroxy-4-methoxybenzophenon).

   Beispielhafte Verbindungen für Dibenzoylmethanderivate:
   Butylmethoxydibenzoylmethan-[1-(4-tert.-butyl)-3-(4-methoxyphenyl)propan-1,3-dion].

   Beispielhafte Verbindungen für Diphenylacrylatderivate:
   Octocrylen-(2-ethylhexyl-2-cyano-3,3'-diphenylacrylat) oder etocrylen-(ethyl-2-cyano-3,3'-diphenylacrylat).

   Beispielhafte Verbindungen für Benzofuranderivate:
   3-(Benzofuranyl)-2-cyanoacrylat, 2-(2-Benzofuranyl)-5-tert.-butylbenzoxazol oder 2-(p-Aminophenyl)benzofuran und insbesondere die Verbindung der Formel 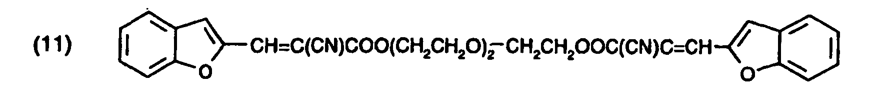 oder

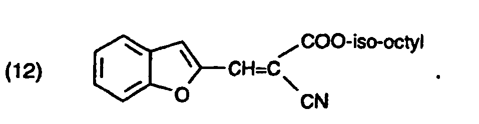

   Beispielhafte Verbindungen für polymere UV-Absorber, die eine oder mehrere silizium-organische Reste enthalten:
   Benzylidenmalonatderivat, insbesondere die Verbindung der Formel 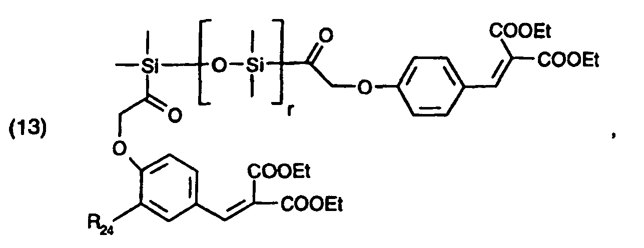
      worin
      - R₂₄: Wasserstoff oder O-Me und
      - r: ungefähr 7; die Verbindung der Formel 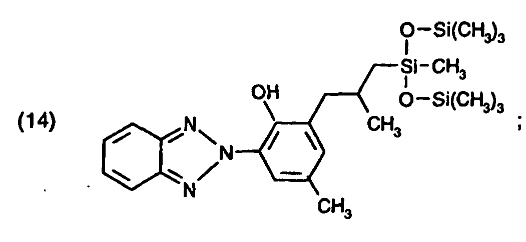
      oder

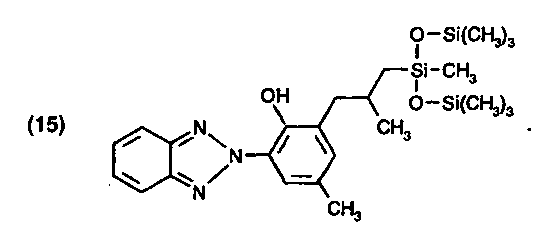

   Beispielhafte Verbindungen für Zimtsäureester:
   Octylmethoxycinnamat (4-Methoxyzimtsäure-2-ethylhexylester), Diethanolaminmethoxycinnamat (Diethanolaminsalz der 4-Methoxyzimtsäure), Isoamyl-p-methoxycinnamat (4-Ethoxyzimtsäure-2-isoamylester), 2,5-Diisopropylmethylcinnamat oder ein Zimtsäureamidoderivat.

   Beispielhafte Verbindungen für Campherderivate:
   4-Methyl-benzylidencampher [3-(4'-Methyl)benzyliden-bornan-2-on], 3-Benzylidencampher (3-Benzyliden-bornan-2-on), Polyacrylamidomethylbenzylidencampher {N-[2(und 4)-2-oxybom-3-yliden-methyl)benryl]acrylamidpolymer}, Trimonium-benzyliden-camphersulfat-[3-(4'-trimethylammonium)-benzyliden-bornan-2-on-methylsulfat], Terephthalydendicampher-Sulfonsäure {3,3'-(1,4-Phenylendimethin)-bis-(7,7-dimethyl-2-oxo-bicyclo-[2.2.1]heptan-1-methansulfonsäure} oder deren Salze, oder Benzylidencampher-Sulfonsäure [3-(4'-sulfo)benzylidenboman-2-on] oder deren Salze.

   Beispielhafte Verbindungen für Trianilino-s-triazinderivate:
   Octyltriazin-[2,4,6-trianilino-(p-carbo-2'-ethyl-1'-oxy)-1,3,5-triazin, sowie die in der US-A-5,332,568, US-A-5,252,323, WO 93/17002 und WO 97/03642 und EP-A-0,517,104 beschriebenen Trianilino-s-triazinderivate; die in der EP-A-0,775,698 beschriebenen Resorcinyl-Triazine, insbesondere die Verbindungen der Formel 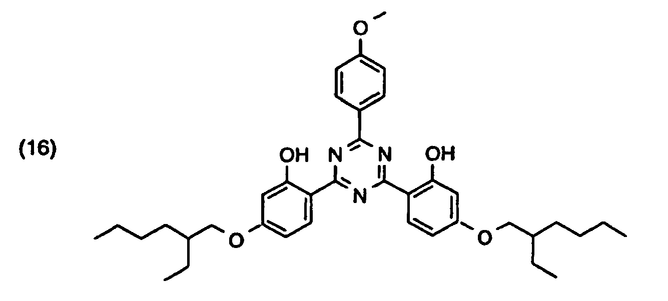 oder

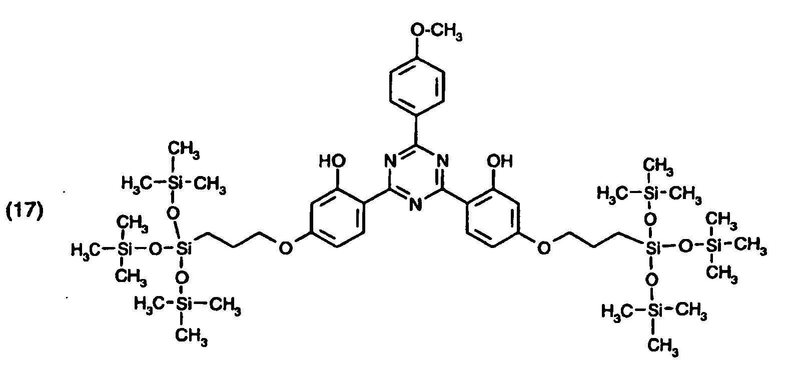

   Beispielhafte Verbindungen für Benzotriazole:
   2-(2-Hydroxy-5-methyl-phenyl)benzotriazol;

   die in der EP-A-0,746,305 offenbarten Benztriazolverbindungen, insbesondere die
   Verbindung der Formel 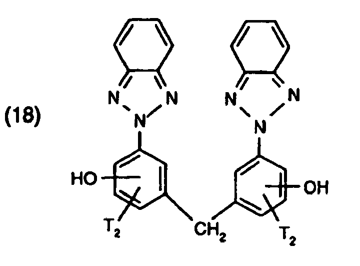
   worin T₂ Wasserstoff; oder C₁-C₈-Alkyl bedeutet;
   - Wirkstoffe gegen Insekten ("repellents): Repellents sind Mittel, die verhindem sollen, dass Insekten die Haut berühren und dort aktiv werden. Sie vertreiben die Tiere und verdampfen langsam. Am häufigsten verwendeter Repellent ist Dethyltolulamid (DEET). Weitere gebräuchliche Repellentien sind in "W. Raab und U. Kindl, "Pflegekosmetik", Gustav-Fischer-Verklag Stuttgart/New York,1991, S.161 zu finden.
   - Wirkstoffe zum Schutz gegen chemische und mechanische Einwirkungen: dazu gehören alle Stoffe, die eine Barriere zwischen der Haut und der äusseren Noxe bilden, wie z.B. Paraffinöle, Silokonöle, Pflanzenöle, PCL-Produkte und Lanolin zum Schutz gegen wässrige Lösungen, Filmbildner, wie Natriumalginat, Triethanolaminalginat; Polyacrylate, Polyvinylalkohol oder Zelluloseether gegen die Einwirkung organischer Lösungsmittel, oder Substanzen auf der Grundlage von Mineralölen, Pflanzenölen oder Sliikonölen als "Schmiermittel" gegen starke mechanische Beanspruchungen der Haut;
   - Feuchthaltesubstanzen: als Feuchthalteregulatoren ("moisturize") finden z.B. folgende Stoffe Verwendung: Natriumlactat, Hamstoff, Alkohole, Sorbit, Glycerin, Propylenglykol, Kollagen, Elastin oder Hyaluronsäure;
   - Wirkstoffe mit keratoplastischem Effekt: Benzoylperoxid, Retinsäure, kolloidaler Schwefel und Resorcin;
   - Antimikrobielle Mittel, wie z.B. Triclosan oder quaternäre Ammoniumverbindungen;
   - Dermal applizierbare ölige oder öllösliche Vitamine oder Vitaminderivate: z.B. Vitamin A (Getinol in Form der freien Säure oder ihrer Derivate), Panthenol, Pantothensäure, Folsäure, und Kombinationen davon, Vitamin E (Tocopherol), F; essentielle Fettsäuren; oder Niacinamid (Nicotinsäureamid);
   - Placentaextrakte auf Vitaminbasis: Wirkstoffzusammensetzungen vor allem mit Vitamin A, C, E, B₂₁ B₁₂, Folsäure und Biotin, Aminosäuren und Fermenten sowie Verbindungen der Spurenelemente Magnesium, Silicium, Phosphor, Calcium, Mangan, Eisen oder Kupfer.
   - Skin Repair Komplexe: erhältlich aus inaktivierten und desintegrierten Kulturen von Bakterien der Bifidusgruppe;
   - Pflanzen und Pflanzenextrakte: wie z.B. Arnika, Aloe, Bartflechte, Efeu, Brennessel, Ginseng, Henna, Kamille, Ringelblume, Rosmarin, Salbei, Schachtelhalm oder Thymian;
   - Tierische Extrakte: wie z.B. Geleé royale, Propolis, Proteine oder Thymusextrakte;
   - dermal applizierbare kosmetische Öle: Neutralöle vom Typ Miglyol 812, Aprikosenkernöl, Avocadoöl, Babassuöl, Baumwollsamenöl, Borretschöl, Distelöl, Erdnussöl, Gamma-Oryzanol, Hagebuttenkemöl, Hanföl, Haselnussöl, Johannisbeersamenöl, Jojobaöl, Kirschkernöl, Lachsöl, Leinöl, Maiskeimöl, Makadamianussöl, Mandelöl, Nachtkerzenöl, Nerzöl, Olivenöl, Pekannussöl, Pfirsichkemöf, Pistazienkernöl, Rapsöl, Reiskeimöl, Rizinusöl, Safloröl, Sesamöl, Sojaöl, Sonnenblumennöl, Teebaumöl, Traubenkemöl oder Weizenkeimöl.

Die Komponenten (a), (b) und (c) können in der erfindungsgemäss eingesetzten Nanodispersion jeweils als Einzelverbindungen oder Mischungen mehrerer verschiedener Einzelkomponenten vorhanden sein.

In der Nanodispersionszusammensetzung ist in der Regel mindestens eine Komponente (a), (b) oder (c) ein in der Kosmetik zur Pflege oder zum Schutz der Haut, Schleimhaut und Haare verwendeter funktioneller Wirkstoff.

Die erfindungsgemäss eingesetzte Nanodispersion enthält einen C₂-C₈-Alkohohl, wie z.B. Ethanol oder Propylenglykol.

Eine Nanodispersion mit den Komponenten (a), (b), (c) und gegebenenfalls (d) zeichnet sich durch günstige Phaseneigenschaften des solubilisierten funktionellen kosmetischen Wirkstoffs aus. So ist bei vorhandener Opaleszenz und Transparenz im Gegenlicht nur an einer äusserst geringen milchigen Trübung zu erkennen, dass die Dispersion noch physikalische Unterschiede gegenüber dem Idealzustand einer echten molekularen Lösung aufweist. Elektronenmikroskopische Abbildungen zeigen, dass eine Population von mehr als 98 % in einer Gaussschen Verteilung als Suspension von Partikeln (Nanopartikel) mit einer Teilchengrösse kleiner als ca. 50 nm, typischerweise < ca. 30 nm vorliegt. Diese Unterschiede gegenüber einer echten Lösung sind aber aufgrund der besonders guten Homogenitätseigenschaften der Dispersion tolerierbar, die beispielsweise an einer überraschend hohen Lagerstabilität, z. B. keine Entmischung nach mehrmonatiger Lagerung bei Temperaturen bis Raumtemperatur (durch Extrapolation zu erwartende Stabilität länger als zwei Jahre), nachweisbar sind.

Laser-Lichtstreumessungen und elektronenmikroskopische Untersuchungen (Cryo-TEM) bestätigen die sehr kleine Grösse und hervorragende Homogenität der in der Nanodispersion vorhandenen Nanopartikel.

Ein weiterer Vorteil der erfindungsgemäss verwendeten Nanodispersionen ist ihre einfache Herstellbarkeit.

Die durch den Anspruch 1 charakterisierten Nanodispersionen werden erfindungsgemäss für kosmetische Endformulierungen verwendet.

Kosmetische Endformulierungen beinhalten die verschiedensten kosmetische Mittel. Insbesondere kommen z.B. die folgenden Mittel in Betracht:
- Mittel zur Hautpflege, wie zB. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder homhautbeseitigende Mittel; .
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Diese aufgezählten Endformulierungen können in den verschiedensten Darreichungsformen vorliegen, wie z.B.
- in Form von flüssige Zubereitungen als einer O/W-Emulsion,
- in Form eines Gels,
- in Form eines Öls, einer Creme, Milch oder Lotion,
- in Form eines Pulvers, eines Lacks, einer Tablette oder Make-Ups,
- in Form eines Stiftes,
- in Form eines Sprays (Spray mit Treibgas oder Pumpspray) oder eines Aerosols,
- in Form eines eines Schaumes, oder
- in Form einer Paste.

Die flüssigen und halbfesten Darreichungsformen enthalten dabei die Nanodispersion mit den Komponenten (a), (b) und (c) in der wässrigen Phase, sowie einen oder mehrere der weiter oben aufgeführten funktionellen kosmetischen Wirkstoffe. Feste Darreichungsformen enthalten die Nanodispersion in der dehydratisierten Form, wobei die Dehydratisierung der Nanodispersion im allgemeinen durch Gefrier- oder Sprühtrocknung in Gegenwart üblicher Hilfsstoffe erfolgt. Für gewisse Endformulierungen ist es von Vorteil, anstelle der Nanodispersion die entsprchende Nanodispersions-Vorphase zu verwenden.

Emulsionen sind heterogene Systeme, die aus zwei miteinander nicht oder nur begrenzt mischbaren Flüssigkeiten (Phasen) bestehen. Die eine liegt dabei in Form von Tröpfchen vor (disperse oder innere Phase), während die andere als Flüssigkeit eine kontinuierliche Phase bildet. Bei einer O/W-Emulsion, deren Grundcharakter durch Wasser geprägt ist, liegen Öltröpfchen feinverteilt in Wasser vor.

Cremes sind gewöhnlich im Bereich von Raum- bis Hauttemperatur streichfähig, während Lotionen oder Milche eher fliessfähig sind.

Gele sind halbfeste, mehr oder weniger transparente Systeme, bei denen der sog.
Gelbildner ein dreidimensionales Netzwerk ausbildet, in dem eine Flüssigkeit immobilisiert ist. Die klaren bis opaquen Hydrogele bestehen primär aus Wasser, wasserlöslichen Substanzen und Verdickem bzw. Gelbildnem. Werden zusätzlich Lipide eingearbeitet, so erhält man die leicht cremig aussehenden Hydrodispersionsgele. Dagegen sind Oleogele frei von Wasser und enthalten Lipide als flüssige Komponenten.

Die kosmetische Endformulierung, die einen oder mehrere der weiter oben aufgezählten Inhaltsstoffe enthält und in den oben erwähnten Darreichungsformen vorliegen kann, enthält die erfindungsgemäss verwendete Nanodispersion vorzugsweise in einer Konzentration von 0,01 bis 100, vorzugsweise von 0,01 bis 20,0, insbesondere 0,05 bis 5 Gew.%.

Diese Endformulierungen bilden einen weiteren Erfindungsgegenstand

Die Endformulierungen enthalten dabei in ihrer wässrigen Phase die Nanodispersion in einer Konzentration von 0,01 bis 20,0, vorzugsweise 0,05 bis 10, und insbesondere von 0,1 bis 5 Gew.%.

Die erfindungsgemässe kosmetische Endformulierung kann auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel wie z.B. Paraben, Antioxidantien, sowie Duft- und Farbstoffe enthalten.

Die Rahmenrezepturen für die kosmetischen Endformulierungen gemäss der Beispiele 14 bis 21 sind gemäss den Angaben in "Kosmetik; Entwicklung, Herstellung und Anwendung kosmetischer Mittel" (Herausg.: W. Umbach, Georg Thieme Verlag Stuttgart, New York) und gemäss den Unterlagen aus "DGK Fortbildungskurs 1998, Entwicklung modemer Hautpflegemittel", erstellt worden.

Die Herstellung der Endformulierungen erfolgt nach den üblichen bekannten und z.B. in der oben angegebenen Literatur beschriebenen Methoden. Bei flüssigen und halbfesten Endformulierungen werden die Nanodispersionen immer in die wässrigen Phase der Endformulierungen eingearbeitet werden. Dazu werden diese in wenigen Anteilen der wässrigen Phase aufgenommen und als Wirkstoffphase als letzte Phase bei der Herstellung einer Formulierung bei ca. 20 bis 30°C zugegeben. Anstelle der Nanodispersion kann auch die entsprechende Nanodispersions-Vorphase der Wasserphase der Enformulierung zugegeben werden. Die Zugabe der Nanodispersions-Vorphase zur Wasserphase erfolgt unter Rühren und vorzugsweise bei einer Temperatur, die im Bereich der jeweiligen Öl/Wasser-Phaseninversionstemperatur (PIT) liegt.

Für feste Endformulierungen ist es vorteilhaft, die dehydratisierte Form der Nanodispersion dem festen Stoffgemisch beizumischen.

Die kosmetischen Endformulierung wird vorzugsweise zur Pflege und zum Schutz der Haut, Schleimhaut oder Haare, und ganz besonders als Sonnenschutzmittel oder sogenanntes "After Sun"-Präparat verwendet.

Weiterhin lassen sich die erfindungsgemäss verwendeten Nanodispersionen auch als Transportvehikel für öllösliche Farbstoffe verwenden.

Einen weiteren Erfindungsgegenstand bildet daher die Verwendung der in Anspruch 1 definierten Nanosdispersion als Carriersystem für öllösliche Farbstoffe.

Es kommen dabei Farbstoffe sowohl synthetischer als auch natürlicher Herkunft in Betracht, die aus allen bekannten Chromophoren aufgebaut sind, wie z.B. Azo-, Azoic-, Anthrachinon-, Caratenoid-, Quinolin-, Xanthen, Diarylmethan-, Triarylmethan-, Stilben-, Indigoid-, Phtalocyanin-, Nitro-Farbstoffe, und alle weiteren bekannten Chromophore, wie sie auch im Colour Index unter Cl 11000 bis Cl 77999 aufgelistet werden.

Von diesen sind besondere solche von Interesse, die in organischen Medien wie Ölen wenigstens zu einem geringem Teil löslich sind. Hierzu gehören zum Beispiel.die Farbstoffe, die als Solvent Dyes oder Disperse Dyes bezeichnet werden, wobei zu den Disperse Dyes auch die Gruppe der ungeladenen direkt aufziehenden Haarfarben zählt, z.B. Derivate des Nitrobenzens oder Nitrodiphenylamins.

Beispiele für erfindungsgemäss einsetzbare Solvent Dyes sind:
Solvent Black 3 (Cas-No.: 4197-25-5); Solvent Black 5 (Cas-No.: 11099-03-9); Solvent Blue 35 (Cas-No.: 12769-17-4); Solvent Green 3; Solvent Green 7 (Cas-No.: 6358-69-6); Solvent Orange 1 (Cas-No.: 2051-85-6); Solvent Red 24 (Cas-No.: 85-83-6); Solvent Red 43 (Cas-No.: 15086-94-9); Solvent Red 48 (Cas-No.: 13473-26-2); Solvent Red 49:1 (Cas-No.: 6373-07-5); Solvent Red 72 (Cas-No.: 596-03-2); Solvent Yellow 44 (CAS No.: 2478-20-8); Solvent Yellow 18 (CAS No.: 6407-76-9).

Beipiele für erfindungsgemäss einsetzbare Disperse Dyes sind:
Disperse Black 9 (CAS No.: 12222-60-4); Disperse Blue 1 (CAS Nos.: 2475-45-8); Disperse Blue 3 (CAS No.: 2475-46-9); Disperse Brown 1 (CAS No.: 23355-64-8); Disperse Orange 3 (CAS No.: 730-40-5); Verbindung der Formel 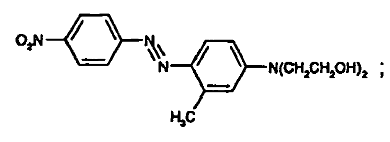 Disperse Violet 1 (CAS-No.: 128-95-0); Disperse Violet 4 (CAS-No.:1220-94-6); Verbindung der Formel

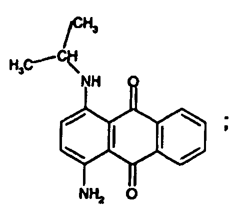

Beispiele für (ungeladene) direkt aufziehende Haarfarben:
HC Blue No. 2 (CAS-No.: 33229-34-4); HC Blue No.4 (Reaktionsprodukt aus N-methyl-1,4-diaminoanthrachinon, Epichlorhydrin und Monoethanolamin); HC Blue No. 5 (CAS-No.: 68478-64-8); Verbindung der Formel 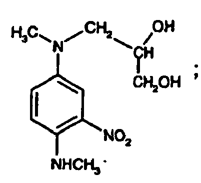 HC Blue No. 7 (Cas-No.: 90817-34-8); HC Blue No. 8 (Cas-No.: 22366-99-0); HC Blue No. 9 (Cas-No.: 114087-42-2); HC Blue No. 10 (Cas-No.: 102767-27-1); HC Blue No. 11 (Cas-No.: 23920-15-2); HC Blue No. 12 (Cas-No.: 132885-85-9); Verbindung der Formel 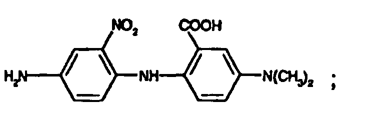 HC Blue No. 14 (Cas-No.: 99788-75-7); HC Orange No. 1 (Cas-No.: 54381-08-7); , HC Orange No 2 (Cas-No.: 85765-48-6), HC Orange No 3 (Cas-No.:81612-54-6); Verbindung der Formel 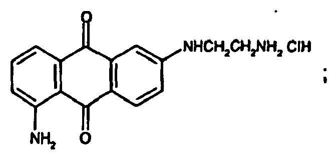 HC Red No. 1 (Cas-No.: 2784-89-6); HC Red No. 3 (Cas-No.: 2871-01-4); HC Red No. 7 (Cas-No.: 24905-87-1); HC Red No. 8 (Cas-No.: 13556-29-1); HC Red No. 9 (Cas-No.:56330-88-2); HC Red No. 10 (Cas-No.: 95576-89-9);, HC Red No. 11 (Cas-No.: 95576-92-4), HC Red No. 13 (Cas-No.: 94158-13-1); ,Verbindung der Formel 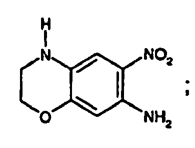 HC Violet No. 1 (Cas-No.: 82576-75-8); Verbindung der Formel 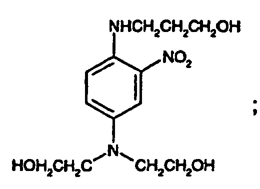 HC Yellow No. 2 (Cas-No.: 4926-55-0); HC Yellow No. 4 (Cas-No.: 59820-43-8); HC Yellow No. 5 (Cas-No.: 56932-44-6); HC Yellow No. 6 (Cas-No.: 104335-00-6); Verbindung der Formel 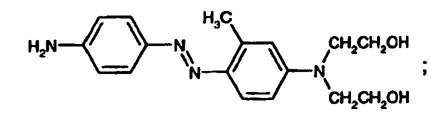 HC Yellow No. 8 (Cas-No.: 66612-11-1); HC Yellow No. 9 (Cas-No.: 86419-69-4); HC Yellow No. 10 (Cas-No.: 109023-83-8); HC Yellow No. 11 (Cas-No.: 73388-54-2); HC Yellow No. 12 (Cas-No.: 59320-13-7); HC Yellow No. 13 (Cas-No.: 10442-83-8); Verbindung der Formel 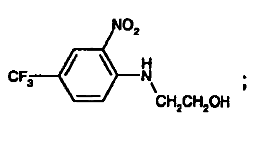 HC Verbindung der Formel 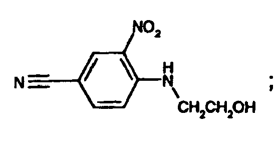 Verbindung der Formel 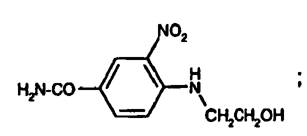 HC Brown No. 1 (Cas-No.: 83803-98-9); HC Brown No. 2 (Cas-No.: 83803-99-0); HC Green No. 1 (Cas-No.: 52136-25-1).

Die erfindungsgemäss verwendeten Nanodispersionen können dabei folgende Funktionen haben:
- als Carrier, um Farbstoffe durch bestimmte Potentialwände in Medien wie die Haut, Haare oder Nägel eindringen zu lassen;
- als Schutz der Farbstoffe vor anderen Inhaltsstoffen einer Formulierung (oder umgekehrt, als Schutz der Formulierung vor einem weiteren Farbstoff) um z.B. Inkompatibilitäten zwischen Formulierung und Farbstoff zu eliminieren. Der Farbstoff ist dann im Kern der Nanodispersion von der restlichen Formulierung getrennt. Dadurch ist es z.B. möglich, vom Farbstoff photo-initiierte Kettenreaktionen mit anderen Inhaltsstoffen zu verhindern;
- um Farbstoffe, die nur in Öl-Phasen löslich sind, in wässrige Systeme einzubringen und zu stabiliseren. Der Farbstoff ist dann im Kern der Nanodispersion (Öl-Phase) gelöst und das Nanodispersions- Partikel wiederum in der wässrigen Phase dispergiert.

In den folgenden Beispielen beziehen sich die Prozentsätze auf das Gewicht. Die Mengen beziehen sich bei den eingesetzten Verbindungen, wenn nicht anders angegeben, auf die Reinsubstanz.

Herstellungsbeispiele für Nanodispersion-Vorphasen

### Beispiel 1: Miglyol 812 Nanodispersion-Vorphase

| | |
|---|---|
| Soja-Lecithin | 17,30 % |
| Oleth-20 | 34,00 % |
| Miglyol 812 | 34,50 % |
| Ethanol | 14,20 % |

Herstellung: Miglyol 812 und Oleth-20 werden unter Erwärmen gemischt. Zu dieser Mischung gibt man das in Ethanol gelöste Soja-Lecithin hinzu und erhält eine homogene, klare Flüssigkeit.

### Beispiel 2: Miglyol 812 Nanodispersion-Vorphase

| | |
|---|---|
| Soja-Lecithin | 17,30% |
| Laneth-20 | 34,00 % |
| Miglyol 812 | 34,50 % |
| Ethanol | 14,20% |

Herstellung: Miglyol 812 und Laneth-20 werden unter Erwärmen gemischt. Zu dieser Mischung gibt man das in Ethanol gelöste Soja-Lecithin hinzu und erhält eine homogene, klare Flüssigkeit.

### Beispiel 3: Miglyol 812 Nanodispersion-Vorphase

| | |
|---|---|
| Soja-Lecithin | 17,30 % |
| Vitamin E Polyethylene Glycol Succinate (Vitamin E TPGS, Eastman) | 34,00 % |
| Miglyol 812 | 34,50 % |
| Ethanol | 14,20 % |

Herstellung: Miglyol 812 und Vitamin E Polyethylene Glycol Succinate werden unter Erwärmen gemischt. Zu dieser Mischung gibt man das in Ethanol gelöste Soja-Lecithin hinzu und erhält eine homogene, klare Flüssigkeit.

Herstellungsbeispiele für Nanodispersionen

### Beispiel 4: Miglyol 812 Nanodispersion

| | |
|---|---|
| Soja-Lecithin | 1.73% |
| Oleth-20 | 3,40 % |
| Miglyol 812 | 3,45 % |
| Ethanol | 1,42% |
| Aqua purificata | ad 100,00 % |

Herstellung: Die Wasser-Phase (z.B. 90 kg) wird unter Rühren (z.B. Magnetrührerwerk) bei 50°C vorgelegt. Die flüssige Nanodispersions-Vorphase des Beispiel 1 (z.B. 10 kg) wird der Wasser-Phase unter Rühren (z.B. mittels eines Magnetrührwerks) zugegeben.

### Beispiel 5: Migylol 812 Nanodispersion

| | |
|---|---|
| Soja-Lecithin | 1,73 % |
| Laneth-20 | 3,40 % |
| Miglyol 812 | 3.45 % |
| Ethanol | 1,42 % |
| Aqua purificata | ad 100,00 % |

Die Herstellung der Nanodispersion erfolgt in zum Beispiel 4 analoger Weise.

### Beispiel 6: Miglyol 812 Nanodispersion

| | |
|---|---|
| Soja-Lecithin | 1,73 % |
| Vitamin E Polyethylene Glycol Succinate (Vitamin E TPGS, Eastman) | 3,40 % |
| Miglyol 812 | 3,45 % |
| Ethanol | 1,42 % |
| Aqua purificata | ad 100,0 % |

Die Herstellung der Nanodispersion erfolgt in zum Beispiel 4 analoger Weise.

### Herstellungsbeispiele für kosmetische Endformulierungen mit Nanodispersionen:

### Beispiel 7: Flüssiges Syndet mit rückfettenden Eigenschaften

| | |
|---|---|
| Fettalkoholethersulfat | 7,5% |
| Rückfetter | 3,0 % |
| Perlglanzgeber | 2,0 % |
| Verdickungsmittel | 1,0 % |
| Nanodispersion gemäss Beispiel 10 | 5,0 % |
| Parfüm | 0,3 % |
| Wasser | ad 100,0 % |

## Patentansprüche

1. Verwendung einer Nanodispersion, enthaltend
(a) 0.1 - 30 Gew-% eines Phospolipid,
(b) 1 - 50 Gew-% polyethoxylierte Fettalkohole, polyethoxylierte Fettsäuren, polyethoxylierte Vitamin E Derivate, polyethoxyliertes Lanolin und dessen Derivate, polyethoxylierte Fettsäurepartialglyceride, polyethoxylierte Alkylphenole, Schwefelsäurehalbester polyethoxylierter Fettalkohole und deren Salze, polyethoxylierte Fettamine und Fettsäureamide, polyethoxylierte Kohlenhydrate, Blockpolymerisate von Ethylenoxid und Propylenoxid
(c) 0.1 - 80 Gew-% eines natürlichen oder eines synthetischen oder eines partialsynthetischen Di- oder Triglycerid, eines Mineralöls, eines Silikonöls, eines Wachs, eines Fettalkohols, eines Guerbet-Alkohols oder dessen Ester, eines lipophilen funktionellen kosmetischen Wirkstoffes oder eine Mischung dieser Stoffe, und
(d) einen C₂-C₈-Alkohol
erhältlich durch
(α) Mischen der Komponenten (a), (b), (c) und (d) mit gewöhnlichen Rührapparaten bis eine homogene, klare Flüssigkeit entsteht, und
(β) Zugabe der im Schritt (α) erhaltenen Flüssigkeit in die Wasserphase, in kosmetischen Endformulierungen.

2. Verwendung nach Anspruch 1, **dadurch gekennzeichnet, dass** der Schritt (α) im wasserfreien Medium durchgeführt wird.

3. Verwendung nach Anspruch 2, **dadurch gekennzeichnet, dass** der Schritt (β) ohne Homogenisierung erfolgt.

4. Verwendung nach einem der Ansprüche 2 bis 3, **dadurch gekennzeichnet, dass** die in der Nanodispersion vorliegenden Partikel einen mittleren Durchmesser von <50 nm aufweisen.

5. Verwendung nach Anspruch 1, **dadurch gekennzeichnet, dass** die Nanodispersion als Komponente
(c) ein Sonnenschutzfilter oder ein fettlösliches Vitamin enthält.

6. Verwendung nach einem der Ansprüche 2 bis 5, **dadurch gekennzeichnet, dass** die Nanodispersion in der Endformulierung in einer Konzentration von 0,01 bis 99 Gew.-% vorhanden ist.

7. Verwendung nach einem der Ansprüche 2 bis 5, **dadurch gekennzeichnet, dass** mindestens eine Komponente (a), (b) oder (c) ein in der Kosmetik zur Pflege oder zum Schutz der Haut, Schleimhaut und Haare verwendeter Inhaltsstoff ist.

8. Kosmetische Endformulierung in Form eines Gels, enthaltend eine Nanodispersion wie in Anspruch 1 definiert.

9. Kosmetische Endformulierung in Form einer Creme, einer Lotion oder Milch, enthaltend eine Nanodispersion wie in Anspruch 1 definiert.

10. Kosmetische Endformulierung in Form eines Stiftes, enthaltend eine Nanodispersion wie in Anspruch 1 definiert.

11. Kosmetische Endformulierung in Form eines Sprays oder Aerosols, enthaltend eine Nanodispersion wie in Anspruch 1 definiert.

12. Kosmetische Endformulierung in Form eines Schaumes, enthaltend eine Nanodispersion wie in Anspruch 1 definiert.

13. Kosmetische Endformulierung in Form einer Paste, enthaltend eine Nanodispersion wie in Anspruch 1 definiert.

14. Kosmetische Endformulierung nach einem der Ansprüche 9 bis 13, **dadurch gekennzeichnet, dass** die Nanodispersion in der wässrigen Phase vorhanden ist.

15. Kosmetische Endformulierung nach einem der Ansprüche 9 bis 14, **dadurch gekennzeichnet, dass** die wäßrige Phase die Nanodispersion in einer Konzentration von 0,01 bis 20 Gew.% enthält.

16. Kosmetische Endformulierung in Form eines Pulvers, eines Lacks, einer Tablette oder Make-Ups, enthaltend eine Nanodispersion wie in Anspruch 1 definiert, **dadurch gekennzeichnet, dass** die Nanodispersion in dehydratisierter Form vorliegt.

## Claims

1. Use of a nanodispersion comprising
(a) 0.1 - 30 % by weight of a phospholipid,
(b) 1 - 50 % by weight of polyethoxylated fatty alcohols, polyethoxylated fatty acids, polyethoxylated vitamin E derivatives, polyethoxylated lanolin and the derivatives thereof, polyethoxylated fatty acid partial glycerides, polyethoxylated alkylphenols, sulphuric acid monoesters of polyethoxylated fatty alcohols and the salts thereof, polyethoxylated fatty amines and fatty acid amides, polyethoxylated carbohydrates, block polymers of ethylene oxide and propylene oxide,
(c) 0.1 - 80 % by weight of a natural or synthetic or partially synthetic di- or triglyceride, a mineral oil, silicone oil, wax, fatty alcohol, guerbet alcohol or ester thereof, a lipophilic functional cosmetic active agent or a mixture of these substances, and
(d) a C₂-C₈ alcohol,
obtainable by
(α) mixing the components (a), (b), (c) and (d) with usual stirring apparatus until a homogeneous clear liquid is obtained, and
(β) adding the liquid obtained in step (α) to the water phase, in cosmetic end formulations.

2. Use according to claim 1, wherein step (α) is carried out in anhydrous medium.

3. Use according to claim 2, wherein step (β) is carried out without homogenization.

4. Use according to any one of claims 2 to 3, wherein the particles in the nanodispersion have an average diameter of < 50 nm.

5. Use according to claim 1, wherein the nanodispersion comprises as component
(c) a sunscreen or a fat-soluble vitamin.

6. Use according to any one of claims 2 to 5, wherein the nanodispersion is present in the end formulation in a concentration of 0.01 to 99 % by weight.

7. Use according to any one of claims 2 to 5, wherein at least one component (a), (b) or (c) is an ingredient used in cosmetics for care-treating or protecting the skin, mucosae and hair.

8. A cosmetic end formulation in the form of a gel, comprising a nanodispersion as defined in claim 1.

9. A cosmetic end formulation in the form of a cream, lotion or milk, comprising a nanodispersion as defined in claim 1.

10. A cosmetic end formulation in the form of a stick, comprising a nanodispersion as defined in claim 1.

11. A cosmetic end formulation in the form of a spray or aerosol, comprising a nanodispersion as defined in claim 1.

12. A cosmetic end formulation in the form of a foam, comprising a nanodispersion as defined in claim 1.

13. A cosmetic end formulation in the form of a paste, comprising a nanodispersion as defined in claim 1.

14. A cosmetic end formulation according to any one of claims 9 to 13, wherein the nanodispersion is present in the aqueous phase.

15. A cosmetic end formulation according to any one of claims 9 to 14, wherein the aqueous phase comprises the nanodispersion in a concentration of 0.01 to 20 % by weight.

16. A cosmetic end formulation in the form of a powder, lacquer, tablet or make-up, comprising a nanodispersion as defined in claim 1, wherein the nanodispersion is present in dehydrated form.

## Revendications

1. Utilisation d'une nanodispersion, renfermant
(a) de 0,1 à 30 % en masse d'un phospolipide,
(b) de 1 à 50 % en masse d'alcools gras polyéthoxylés, d'acides gras polyéthoxylés, de dérivés polyéthoxylés de la vitamine E, de lanoline polyéthoxylée et de ses dérivés, de glycérides partiels d'acides gras polyéthoxylés, d'alkylphénols polyéthoxylés, d'hémi-esters de l'acide sulfurique et d'alcools gras polyéthoxylés et leurs sels, d'amines grasses et d'amides gras polyéthoxylés, d'hydrocarbures polyéthoxylés, de polymères à blocs d'oxyde d'éthylène et d'oxyde de propylène,
(c) de 0,1 à 80 % en masse d'un di- ou triglycéride naturel ou synthétique ou partiellement synthétique, d'une huile minérale, d'une huile silicone, d'une cire, d'un alcool gras, d'un alcool de Guerbet ou de ses esters, d'un principe actif cosmétique fonctionnel lipophile ou d'un mélange de ces matières, et
(d) d'un alcool en C₂-C₈,
que l'on peut obtenir par
(α) mélange des composants (a), (b), (c) et (d) en utilisant des appareils de mélange courants jusqu'à l'obtention d'un liquide limpide, homogène, et
(β) ajout du liquide obtenu à l'étape (α) dans la phase aqueuse, aux formules cosmétiques finies.

2. Utilisation selon la revendication 1, **caractérisée en ce que** l'étape (α) est mise en oeuvre dans un milieu non aqueux.

3. Utilisation selon la revendication 2, **caractérisée en ce que** l'étape (β) est mise en oeuvre sans homogénéisation.

4. Utilisation selon l'une des revendications 2 à 3, **caractérisée en ce que** les particules contenues dans la nanodispersion présentent un diamètre moyen <50 nm.

5. Utilisation selon la revendication 1, **caractérisée en ce que** la nanodispersion renferme en tant que composant
(c) un écran solaire ou une vitamine liposoluble.

6. Utilisation selon l'une des revendications 2 à 5, **caractérisée en ce que** la nanodispersion dans la formule finie est présente en une concentration de 0,01 à 99 % en masse.

7. Utilisation selon l'une des revendications 2 à 5, **caractérisée en ce que** au moins un composant (a), (b) ou (c) est une matière contenue utilisée en cosmétique pour les soins ou la protection de la peau, des muqueuses et des cheveux.

8. Formule cosmétique finie sous forme d'un gel, renfermant une nanodispersion telle que définie à la revendication 1.

9. Formule cosmétique finie sous forme d'une crème, d'une lotion ou d'un lait, renfermant une nanodispersion telle que définie à la revendication 1.

10. Formule cosmétique finie sous forme d'un stick, renfermant une nanodispersion telle que définie à la revendication 1.

11. Formule cosmétique finie sous forme d'un spray ou aérosols, renfermant une nanodispersion telle que définie à la revendication 1.

12. Formule cosmétique finie sous forme d'une mousse, renfermant une nanodispersion telle que définie à la revendication 1.

13. Formule cosmétique finie sous forme d'une pâte, renfermant une nanodispersion telle que définie à la revendication 1.

14. Formule cosmétique finie selon l'une des revendications 9 à 13, **caractérisée en ce que** la nanodispersion est présente dans la phase aqueuse.

15. Formule cosmétique finie selon l'une des revendications 9 à 14, **caractérisée en ce** la phase aqueuse renferme la nanodispersion en une concentration de 0,01 à 20 % en masse.

16. Formule cosmétique finie sous forme d'une poudre, d'une laque, d'un comprimé ou d'un fard, renfermant une nanodispersion telle que définie à la revendication 1, **caractérisée en ce que** la nanodispersion est présente sous forme déshydratée.
